# EUROPEAN PATENT SPECIFICATION

(11) **EP 3 716 832 B1**
(45) Date of publication and mention of the grant of the patent: **30.04.2025**
(21) Application number: 18882466.8
(22) Date of filing: 30.11.2018
(51) Int. Cl.: A47K 10/24, A47K 10/36, A47K 5/06, A61L 9/12, A61L 9/14, A47K 10/32, A47K 10/38, G05B 19/042

(54) **DISPENSING SYSTEM**
AUSGABESYSTEM
SYSTÈME DE DISTRIBUTION

(30) Priority: 30.11.2017 US 201762592743 P
(43) Date of publication of application: 07.10.2020
(73) Proprietor: Kimberly-Clark Worldwide, Inc., Neenah, Wisconsin 54956 (US)
(72) Inventor: WILLIAMS, Frederick J., Jr., Cumming Georgia 30040 (US)
(74) Representative: Dehns
(86) International application number: PCT/US2018/063411
(87) International publication number: WO 2019/108996

(56) References cited:
- AU-A1- 2014 227 493
- KR-B1- 100 765 153
- US-A- 5 608 643
- US-A1- 2005 171 634
- US-A1- 2005 171 634
- US-A1- 2012 013 470
- US-A1- 2016 364 685
- US-B1- 6 360 181

## Description

### BACKGROUND

This disclosure relates to managing servicing dispensers through a centralized controller in the vicinity of the dispensers.

Systems dispensing consumable products are ubiquitous in many environments today. For example, hand towel dispensers are commonplace in many private, semi-private and public washrooms, work areas, food processing stations and kitchens. Monitoring and refilling such dispensers can be a time consuming and laborious endeavor requiring, in some scenarios, that an attendant or building maintenance team member routinely check each dispenser and refill or service as needed. This process inevitably results in checking a dispenser and determining that no refill is required, resulting in an unnecessary visit to the dispenser, which leads to building management inefficiencies and additional costs.

US 2005/0171634 A1 discloses a dispensing system having the features of the preamble of claim 1. AU 2014 227 493 A1 discloses a system for monitoring a washroom including product dispensers.

### SUMMARY

In general, the subject matter of this specification relates to managing the servicing process of dispensers and other devices for efficient maintenance.

One aspect of the present invention provides a dispensing system in accordance with claim 1.

Another aspect of the present invention provides a method in accordance with claim 10.

Particular embodiments of the subject matter described in this specification can be implemented so as to realize one or more of the following advantages. For example, products (e.g., hand towels or hand soap) from all dispensers are not generally consumed at the same rate (e.g., dispensers nearest the exit may be depleted sooner or at a faster rate than other dispensers in the washroom at less convenient locations). Thus it often happens that some dispensers need to be refilled during regularly scheduled service visits as they are empty or near empty while other dispensers, which may have significant product remaining, don't need to be refilled at that time. However, when service personnel enter a washroom they do not always know which dispensers need servicing (e.g., need to be refilled with product or have a malfunction) until they open and/or try to operate the dispenser. Thus, it may be that a washroom includes ten dispensers and only one needs to be serviced. Unfortunately, the service attendants do not know which, if any, need to be serviced so they check all dispensers, which can be a time consuming process.

To avoid unnecessarily checking dispensers that do not need to be serviced, a controller in the washroom, which communicates with the dispensers, enters a service mode (e.g., as caused by the service attendant) that audibly signals to the service attendant which dispenser(s) need to be serviced and the cause for the servicing (e.g., refill or to correct a malfunction). This reduces or eliminates the need to visit dispensers that don't need to be serviced and, for those that do, provides an indication of the particular service need. Thus the controller informs service attendants which dispensers to service, which, in turn, leads to improvements in the operational efficiencies in the care of the washroom and reduces costs (e.g., by reducing unnecessary service visits).

The details of one or more implementations of the subject matter described in this specification are set forth in the accompanying drawings and the description below. Other features, aspects, and advantages of the subject matter will become apparent from the description, the drawings, and the claims.

### DESCRIPTION OF DRAWINGS

Fig. 1 is a block diagram of an example environment in which a dispensing system can be implemented.
Fig. 2 is a block diagram of an example controller.
Fig. 3 is a flow chart of an example process for managing servicing dispensers.

Like reference symbols in the various drawings indicate like elements.

### DETAILED DESCRIPTION

The present disclosure generally relates to managing the servicing of dispensers (e.g., hygienic dispensers). In some implementations, the dispensing system includes a controller located in a hygiene environment, e.g., a washroom, that communicates with the dispensers in that environment to determine the state or status of the dispensers. Such state or status information may include whether the dispenser is malfunctioning, has a low battery, the amount of consumable product remaining or used in the dispenser, whether the dispenser is low on consumable product, etc.

When the washroom is scheduled or about to be serviced, the controller can enter a service mode in which it audibly and, optionally, visually communicates to a service technician which dispensers need to be serviced and, optionally, the nature of such service need. For example, when in the service mode, the controller verbally announces (e.g., speak) the identity of the dispensers that need to be serviced (e.g., the paper towel dispenser next to the sink, the bath tissue dispenser in the middle stall, or dispenser XYZ) and the particular type of service to be performed (e.g., low paper, low battery, paper jam) and the controller may cause lights on the dispensers that need to be serviced to flash or otherwise illuminate. Thus the service technician only needs to focus on the dispensers with a service need and can bypass the others.

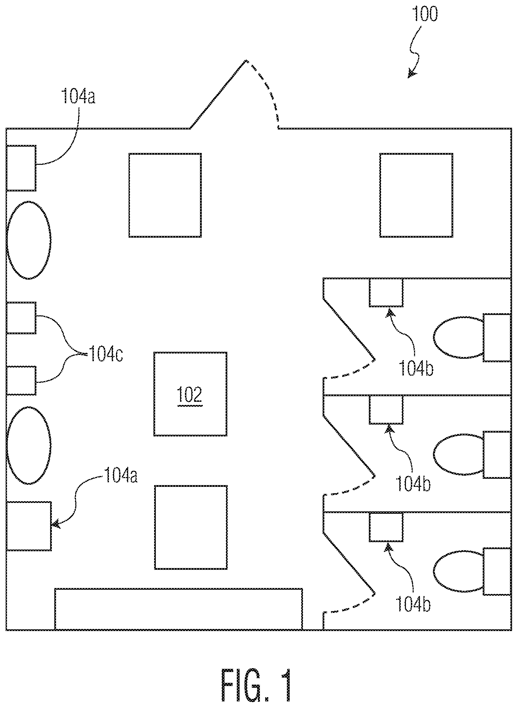

In some implementations, the controller can communicate the state or status of some or all of the dispensers (including the ones without an immediate service need) so that the service technician can determine if any proactive servicing or maintenance is warranted, in addition to the dispensers with immediate service needs. The operation of such a dispensing system is described in more detail below with reference to Fig. 1.

Fig. 1 is a block diagram of an example environment in which a dispensing system 100 can be implemented. The dispensing system 100 includes a plurality of dispensers 104 and a controller 102 or data processing system 102. The environment can include, for example, a semi-private or public washroom or break room or another space in which the dispensers 104 are located. The dispensers 104 can include, for example, hand towel dispensers 104a, bath tissue dispensers 104b, hand soap (or other cleansing) dispensers 104c, air care and facial care dispensers (not pictured), surface cleaning, sanitizing or disinfecting dispensers (not pictured) including for toilets or urinals, and/or the like. These types of dispensers 104 generally dispense consumable hygiene products, which are products intended to promote good hygiene or sanitation such as by cleaning or sanitizing a user and/or a surface. A dispenser 104, more generally, is a device that holds consumable product and dispenses the consumable product in response to a stimulus, e.g., an environmental stimulus (e.g., light/darkness), at pre-determined (e.g., programmatically set) intervals or by manual user actuation such as pulling an exposed portion of the consumable product or via a pumping-type process (e.g., for some manual soap dispensers).

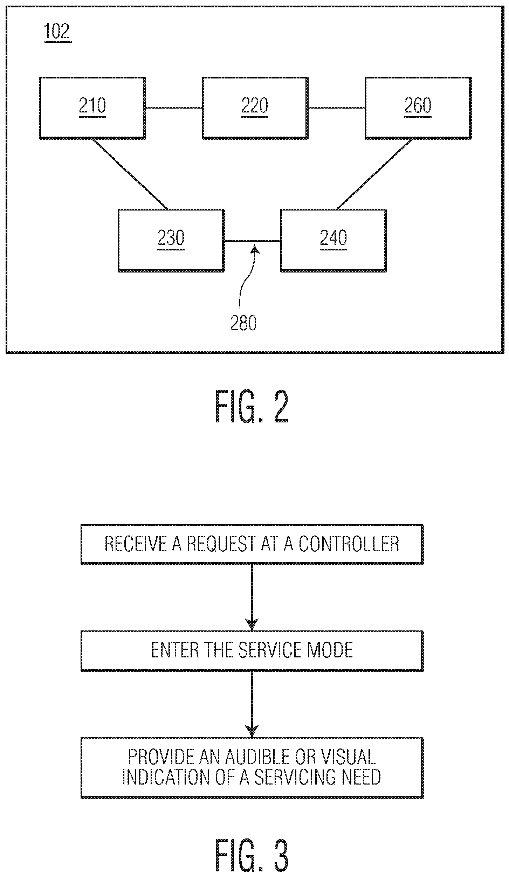

As described above, the dispensing system 100 includes a controller 102. The controller 102 can communicate with the dispensers 104 across wireless or wired channels, or some combination thereof. For example, in some implementations, the controller 102 includes a transceiver and microprocessor to facilitate such communications. The controller 102 is described in more detail below in reference to Fig. 2. In some implementations, as described above, the controller 102 communicates with the dispensers 104 (and other devices such as mobile devices) through one or more wireless communication channels such as, for example, the BLUETOOTH protocol, mesh-based (e.g., ZIGBEE) protocols, and/or through a WAN or LAN.

In some implementations, the controller 102 receives (or requests) from the dispensers 104 product usage information, product remaining information (e.g., the number of dispenses since the last refill or report from the dispenser 104) and/or state/status information (e.g., fault conditions such as jams or low battery alerts). The controller 102 can store this data for later access and use ("Dispenser Condition Information"). The dispensers 104 can send the reports/information, for example, periodically (e.g., hourly or daily or after certain dispenser events such as after each dispense, a set number of dispenses or a fault condition like a jam), upon the controller's request and/or upon a low product condition (e.g., only 10% of the product remains). The reports can include time stamps indicating the date and time of each dispense and the identity of the dispenser (e.g., a unique identifier of the dispenser).

As described above, the controller 102 is located in or proximate to the washroom in which the dispensers 14 it manages are located, as shown in Fig. 1. The controller 102 is remote to all or at least some of these dispensers 104. For example, the controller 102 can be located on the ceiling of the washroom (e.g., to provide a good communication path to the dispensers 104) with the dispensers 104 being located on walls, in stalls, on countertops, among other places.

Fig. 2 is block diagram of an example controller 102. The controller 102 can include a processor 210, a memory 220, a storage device 230, and an input/output device 240. Each of the components 210, 220, 230, and 240 can, for example, be interconnected using a system bus 280. The processor 210 is capable of processing instructions for execution within the controller 102. In one implementation, the processor 210 is a single-threaded processor. In another implementation, the processor 210 is a multi-threaded processor. The processor 210 is capable of processing instructions stored in the memory 220 or on the storage device 230.

The memory 220 stores information within the controller 102. In one implementation, the memory 220 is a computer-readable medium. In one implementation, the memory 220 is a volatile memory unit. In another implementation, the memory 220 is a non-volatile memory unit or a combination of volatile and non-volatile memory.

The storage device 230 is capable of providing mass storage for the controller 102. In one implementation, the storage device 230 is a computer-readable medium.

The input/output device 240 provides input/output operations for the controller 102. In one implementation, the input/output device 240 can include one or more of a network interface device(s), e.g., an Ethernet card, a serial communication device, e.g., and RS-232 port, a wireless interface device or a transceiver, e.g., an 802.11 card, BLUETOOTH interface, ZIGBEE interface.

The controller 102 has a speaker and can also include other communication device(s) 260, e.g., display device, lights, microphone, speakers, to receive input data or information and/or send or communicate output data or information or indications to other input/output devices or users, e.g., service attendants.

In some implementations, the controller 102 is separate and distinct from each dispenser 104. However, in other implementations, the controller 102 may be integral to a dispenser 104, e.g., part of the same device such that the integrated dispenser-controller functions both a dispenser 104 and the controller 102. Further, in some implementations, there may be multiple controllers 102 managing a washroom, e.g., multiple controllers 102 may be required as one controller 102 may not be able to communication with all dispensers 104 depending on the wireless propagation characteristics of the washroom. In this scenario, the multiple controllers 102 may communicate with each other with one being the master controller 102 and the others secondary controllers 102 or each controller 102 may independently manage its own set of dispensers 104.

The controller 102 has a service mode and an operation mode. The operation mode is a mode in which the controller 102 routinely communicates with the dispensers (e.g., receiving Dispenser Condition Information) to, monitor and manage dispenser operation, as described above. The service mode is a mode in which the controller 102 leaves the operation mode (or runs with it simultaneously) to aid service attendants in servicing the washroom by providing (e.g., through itself or by causing another device to do so) audible indications and, optionally, visual indications identifying which of the dispensers 104 in the washroom need to be serviced based on the Dispenser Condition Information and, optionally, what that (those) service need(s) is (are). Among others, based on or derived from the Dispenser Condition Information, the servicing needs can include (a) low or no consumable hygiene product remaining status (e.g., indicating that so the service attendant should refill the dispenser 104), (b) an electrical or mechanical malfunction (e.g., a paper jam) to alert the attendant that the dispenser is not operating properly and that remedial action is needed and (c) a low battery condition indicating that the battery in the dispenser needs to be changed.

The controller 102 can enter the service mode in one or more ways. For example, the controller 102 enters the service mode in response to a verbal command as received through the microphone 260 of the controller 102 and/or in response to detecting the presence of an attendant in the washroom, e.g., via detection of a location beacon on a service attendant or the attendant accessing a keypad. The controller 102 may also or alternatively enter the service mode in response to receipt of a communication from a device, e.g., a service attendant issues an instruction through an application (e.g., integrated with an API on the controller 102) on his/her mobile phone or device to the controller 102 to cause the controller 102 to enter the service mode. Or enter the service mode at predetermined times, e.g., at times corresponding to the service schedule for the washroom or as otherwise programmatically set by a system administrator or user.

Once in the service mode, the controller 102 indicates dispenser service needs to the service attendant by using its speaker 260 to verbally announce the name or identifier of the dispenser 104 that needs servicing and the nature of the particular serving need for that dispenser 104, e.g., refill, paper jam, etc. In some implementations, the controller 102 can additionally or alternatively identify a dispenser 104 that needs servicing by describing to the service attendant its location in the washroom and/or dispenser type, e.g., based on data pre-programmed into the controller 102.

In some implementations, the controller 102 can indicate dispenser service needs to the service attendant, for example, by wirelessly communicating the name or identifier of the dispenser that needs servicing and, optionally, the nature of the particular serving need for that dispenser 104. For example, the controller 102 can send this information to a handheld device of the service attendant for display. Additionally or alternatively, the controller 102 can include a light and use the light to shine on the dispenser 104 that needs serviced by based on accessing a stored, preprogrammed map of the washroom with dispenser location to determine where to shine the light.

In some implementations, as described above, the controller 102 can send an instruction to the dispenser 104 to cause the dispenser 104 with a service need to visually (e.g., flashing a light or actuating a dispense or partial dispense cycle) or audibly (e.g., beeping) indicate it needs to be serviced. In some implementations, the controller 102 can additionally verbally identify the dispenser 104 and/or its servicing need while the dispenser 104 also indicates it needs to be serviced.

In some implementations, the controller 102 can indicate the dispensers' service needs in a batch form, e.g., one immediately after the other, or one-at-a-time and wait to indicate the next dispenser/service need until either the current dispenser 104 has been serviced, after a prescribed period of time (e.g., as set by an administrator), or as otherwise requested to by the service attendant. At any point, in response to a request from the service attendant, e.g., through the attendant's handheld device or verbally, the controller 102 can repeat any or all of the servicing needs.

In some implementations, after a dispenser 104 has been serviced the controller 102 can receive input from the service technician of such (or at the next report received from the dispenser 104) and record that service event as having been completed. In response to receiving input that a dispenser 104 has been serviced, the controller 102 can provide a positive reinforcement communication to the attendant, for example, by playing music or speaking a positive message.

The controller 102 can re-enter the operation mode after all dispensers 104 have been serviced, after a pre-determined period or in response to instructions from the service attendant.

Fig. 3 is a flow chart of an example process for managing dispensers 104. The dispensing system 100 performs the steps described with reference to Fig. 3. In accordance with the present invention, the steps include providing an audible indication of a servicing need by verbally speaking both the servicing need of a dispenser 104 and its identifier through the speaker 260 of the controller 102.

Although the above description has focused on serving dispensers in a washroom, the technology is applicable to other environments in which a central controller is managing remote devices that need to be serviced.

Implementations or aspects of the subject matter and the operations described in this specification can be implemented in digital electronic circuitry, or in computer software, firmware, or hardware, including the structures disclosed in this specification and their structural equivalents, or in combinations of one or more of them. Implementations of the subject matter described in this specification can be implemented as one or more computer programs, i.e., one or more modules of computer program instructions, encoded on computer storage medium for execution by, or to control the operation of, data processing apparatus. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus.

A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., multiple CDs, disks, or other storage devices).

The operations described in this specification can be implemented as operations performed by a data processing apparatus or system on data stored on one or more computer-readable storage devices or received from other sources.

The term data processing apparatus or data processing system encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communication network.

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing actions in accordance with instructions and one or more memory devices for storing instructions and data. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

Implementations of the subject matter described in this specification may, in some implementations, be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

While this specification contains many specific implementation details, these should not be construed as limitations on the scope of any inventions or of what may be claimed, but rather as descriptions of features specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

The separation of various system components in the embodiments described above should not be understood as requiring such separation in all embodiments, and it should be understood that the described program components and systems can generally be integrated together in a single software product or packaged into multiple software products.

## Claims

1. A dispensing system comprising:
a plurality of dispensers (104) each configured to be located in a same washroom and dispense a respective consumable hygiene product, wherein each of the plurality of dispensers (104) has an identifier; and
a controller (102) configured to be located in the washroom, remote to the plurality of dispensers (104) and having a service mode and an operation mode different than the service mode, the controller (102) configured to receive a request to enter into the service mode and to enter into the service mode in response to receiving the request, wherein the controller (102) has a speaker (260),
wherein, when in the service mode, the controller (102) is configured cause an audible indication of a servicing need of at least one of the plurality of dispensers (104), and wherein the servicing need is one of the at least one of the plurality of dispensers (104) having:
low or no consumable hygiene product remaining,
an electrical or mechanical malfunction, and
a low battery condition,
**characterized in that**:
when in the service mode, the controller (102) is configured to verbally speak both the servicing need of the at least one of the plurality of dispensers (104) and its identifier.

2. The dispensing system of claim 1, wherein the plurality of dispensers (104) comprises at least one of a bath tissue dispenser, a paper towel dispenser, a soap dispenser, an air freshener dispenser, and a sanitizer dispenser.

3. The dispensing system of claim 1, wherein the controller (102) is configured to enter the service mode in response to a verbal command.

4. The dispensing system of claim 1, wherein the controller (102) is configured to enter the service mode in response to detecting the presence of a location beacon on a service attendant.

5. The dispensing system of claim 1, wherein the controller (102) is configured to enter the service mode in response to receipt of a communication from a wireless device initiated by a service attendant.

6. The dispensing system of claim 1, wherein the controller (102), when in the service mode, is configured to send the servicing need and the identifier to a wireless device of a service attendant.

7. The dispensing system of claim 1, wherein the controller (102), when in the service mode, is configured to communicate with the at least one of the plurality of dispensers (104) to cause the at least one of the plurality of dispensers (104) to illuminate a light on the at least one of the plurality of dispensers (104).

8. The dispensing system of claim 1, wherein the controller (102), when in the service mode, is configured to communicate with the at least one of the plurality of dispensers (104) to cause the at least one of the plurality of dispensers (104) to issue a visual cue indicting it has the servicing need.

9. The dispensing system of claim 1, wherein the electrical or mechanical malfunction is a jam preventing the at least one of the plurality of dispensers (104) from dispensing or from properly dispensing.

10. A method comprising:
receiving a request at a controller (102) to enter into a service mode, wherein the controller (102) is located in a hygiene area and has an operation mode different than the service mode wherein the controller (102) has a speaker (260);
entering the service mode based on the request;
in the service mode, providing an audible indication of a servicing need of a dispenser (104), wherein the dispenser (104) dispenses a consumable hygiene product, is located in the hygiene area and has an identifier; and
wherein the servicing need is one of low or no consumable hygiene product remaining, an electrical or mechanical malfunction, and a low battery condition,
**characterized in that**:
when in the service mode, providing an audible indication of a servicing need comprises verbally speaking both the servicing need of the at least one of the plurality of dispensers (104) and its identifier through the speaker (260) of the controller (102).

11. The method of claim 10, further comprising the controller (102) communicating a message to the dispenser (104) to cause the dispenser (104) to issue a visual cue of the servicing need.

12. The method of claim 11, wherein the visual cue is an illumination of a light on the dispenser.

13. The method of claim 10, wherein receiving a request at a controller (102) to enter into a service mode comprises receiving a message from a wireless device of a service attendant instructing the controller (102) to enter the service mode.

14. The method of claim 10, wherein receiving a request at a controller (102) to enter into a service mode comprises receiving a spoken command instructing the controller (102) to enter the service mode.

## Patentansprüche

1. Ausgabesystem, umfassend:
eine Vielzahl von Spendern (104), die jeweils so konfiguriert sind, dass sie sich in einem gleichen Waschraum befinden und ein jeweiliges Hygieneverbrauchsprodukt ausgeben, wobei jeder der Vielzahl von Spendern (104) eine Kennung aufweist; und
eine Steuerung (102), die so konfiguriert ist, dass sie sich in dem Waschraum, entfernt von der Vielzahl von Spendern (104), befindet und einen Wartungsmodus und einen Betriebsmodus, der sich von dem Wartungsmodus unterscheidet, aufweist, wobei die Steuerung (102) konfiguriert ist, eine Anforderung zum Eintreten in den Wartungsmodus zu empfangen und als Reaktion auf das Empfangen der Anforderung in den Wartungsmodus einzutreten, wobei die Steuerung (102) einen Lautsprecher (260) aufweist,
wobei die Steuerung (102), wenn sie sich in dem Wartungsmodus befindet, so konfiguriert ist, dass sie eine hörbare Angabe eines Wartungsbedarfs mindestens eines der Vielzahl von Spendern (104) bewirkt, und wobei der Wartungsbedarf darin besteht, dass der mindestens eine der Vielzahl von Spendern (104) eines der Folgenden aufweist:
wenig oder kein verbleibendes Hygieneverbrauchsprodukt,
eine elektrische oder mechanische Fehlfunktion, und
einen niedrigen Batteriestand,
**dadurch gekennzeichnet, dass**:
die Steuerung (102), wenn sie sich in dem Wartungsmodus befindet, konfiguriert ist, sowohl den Wartungsbedarf des mindestens einen der Vielzahl von Spendern (104) als auch dessen Kennung verbal zu äußern.

2. Ausgabesystem nach Anspruch 1, wobei die Vielzahl von Spendern (104) mindestens einen von einem Papiertuchspender, einem Papierhandtuchspender, einem Seifenspender, einem Lufterfrischerspender und einem Desinfektionsmittelspender umfasst.

3. Ausgabesystem nach Anspruch 1, wobei die Steuerung (102) konfiguriert ist, als Reaktion auf einen verbalen Befehl in den Wartungsmodus einzutreten.

4. Ausgabesystem nach Anspruch 1, wobei die Steuerung (102) konfiguriert ist, als Reaktion auf ein Detektieren des Vorhandenseins einer Standortbake an einem Wartungsbediensteten in den Wartungsmodus einzutreten.

5. Ausgabesystem nach Anspruch 1, wobei die Steuerung (102) konfiguriert ist, als Reaktion auf den Empfang einer Kommunikation von einer Drahtlosvorrichtung, die durch einen Wartungsbediensteten initiiert wird, in den Wartungsmodus einzutreten.

6. Ausgabesystem nach Anspruch 1, wobei die Steuerung (102), wenn sie sich in dem Wartungsmodus befindet, konfiguriert ist, den Wartungsbedarf und die Kennung an eine Drahtlosvorrichtung eines Wartungsbediensteten zu senden.

7. Ausgabesystem nach Anspruch 1, wobei die Steuerung (102), wenn sie sich in dem Wartungsmodus befindet, konfiguriert ist, mit dem mindestens einen der Vielzahl von Spendern (104) zu kommunizieren, um zu bewirken, dass der mindestens eine der Vielzahl von Spendern (104) eine Leuchte an dem mindestens einen der Vielzahl von Spendern (104) aufleuchten lässt.

8. Ausgabesystem nach Anspruch 1, wobei die Steuerung (102), wenn sie sich in dem Wartungsmodus befindet, konfiguriert ist, mit dem mindestens einen der Vielzahl von Spendern (104) zu kommunizieren, um zu bewirken, dass der mindestens eine der Vielzahl von Spendern (104) einen visuellen Hinweis ausgibt, der angibt, dass er den Wartungsbedarf aufweist.

9. Ausgabesystem nach Anspruch 1, wobei die elektrische oder mechanische Fehlfunktion ein Stau ist, der eine Ausgabe oder ordnungsgemäße Ausgabe seitens des mindestens einen der Vielzahl von Spendern (104) verhindert.

10. Verfahren, umfassend:
Empfangen einer Anforderung an einer Steuerung (102) zum Eintreten in einen Wartungsmodus, wobei sich die Steuerung (102) in einem Hygienebereich befindet und einen Betriebsmodus aufweist, der sich von dem Wartungsmodus unterscheidet, wobei die Steuerung (102) einen Lautsprecher (260) aufweist;
Eintreten in den Wartungsmodus basierend auf der Anforderung;
in dem Wartungsmodus, Bereitstellen einer akustischen Angabe eines Wartungsbedarfs eines Spenders (104), wobei der Spender (104) ein Hygieneverbrauchsprodukt ausgibt, sich in dem Hygienebereich befindet und eine Kennung aufweist; und
wobei der Wartungsbedarf eines von wenig oder keinem verbleibenden Hygieneverbrauchsprodukt, einer elektrischen oder mechanischen Fehlfunktion oder einem niedrigen Batteriestand ist, **dadurch gekennzeichnet, dass**:
wenn in dem Wartungsmodus, das Bereitstellen einer hörbaren Angabe eines Wartungsbedarfs ein verbales Äußern sowohl des Wartungsbedarfs des mindestens einen der Vielzahl von Spendern (104) als auch seiner Kennung durch den Lautsprecher (260) der Steuerung (102) umfasst.

11. Verfahren nach Anspruch 10, ferner umfassend, dass die Steuerung (102) eine Nachricht an den Spender (104) kommuniziert, um zu bewirken, dass der Spender (104) einen visuellen Hinweis auf den Wartungsbedarf ausgibt.

12. Verfahren nach Anspruch 11, wobei der visuelle Hinweis ein Aufleuchtenlassen einer Leuchte an dem Spender ist.

13. Verfahren nach Anspruch 10, wobei das Empfangen einer Anforderung an einer Steuerung (102) zum Eintreten in einen Wartungsmodus ein Empfangen einer Nachricht von einer Drahtlosvorrichtung eines Wartungsbediensteten umfasst, die die Steuerung (102) anweist, in den Wartungsmodus einzutreten.

14. Verfahren nach Anspruch 10, wobei das Empfangen einer Anforderung an einer Steuerung (102) zum Eintreten in einen Wartungsmodus ein Empfangen eines gesprochenen Befehls umfasst, der die Steuerung (102) anweist, in den Wartungsmodus einzutreten.

## Revendications

1. Système de distribution comprenant :
une pluralité de distributeurs (104) configurés chacun pour être situés dans une même salle de bains et distribuer un produit d'hygiène consommable respectif, dans lequel chacun de la pluralité de distributeurs (104) a un identifiant ; et
un dispositif de commande (102) configuré pour être situé dans la salle de bains, à distance de la pluralité de distributeurs (104) et ayant un mode d'entretien et un mode de fonctionnement différent du mode d'entretien, le dispositif de commande (102) étant configuré pour recevoir une demande pour entrer dans le mode d'entretien et pour entrer dans le mode d'entretien en réponse à la réception de la demande, dans lequel le dispositif de commande (102) a un haut-parleur (260),
dans lequel, lorsqu'il est dans le mode d'entretien, le dispositif de commande (102) est configuré pour provoquer une indication audible d'un besoin d'entretien de l'au moins un de la pluralité de distributeurs (104), et dans lequel le besoin d'entretien est l'un parmi l'au moins un de la pluralité de distributeurs (104) ayant :
peu ou pas de produit d'hygiène consommable restant,
un dysfonctionnement électrique ou mécanique, et
un état de batterie faible,
**caractérisé en ce que** :
lorsqu'il est en mode d'entretien, le dispositif de commande (102) est configuré pour énoncer verbalement à la fois le besoin d'entretien de l'au moins un de la pluralité de distributeurs (104) et son identifiant.

2. Système de distribution selon la revendication 1, dans lequel la pluralité de distributeurs (104) comprend l'au moins un parmi un distributeur de papier hygiénique, un distributeur d'essuie-mains en papier, un distributeur de savon, un distributeur de désodorisant et un distributeur de désinfectant.

3. Système de distribution selon la revendication 1, dans lequel le dispositif de commande (102) est configuré pour entrer dans le mode d'entretien en réponse à un ordre vocal.

4. Système de distribution selon la revendication 1, dans lequel le dispositif de commande (102) est configuré pour entrer dans le mode d'entretien en réponse à la détection de la présence d'une balise de localisation sur un préposé à l'entretien.

5. Système de distribution selon la revendication 1, dans lequel le dispositif de commande (102) est configuré pour entrer dans le mode d'entretien en réponse à la réception d'une communication provenant d'un dispositif sans fil lancée par un préposé à l'entretien.

6. Système de distribution selon la revendication 1, dans lequel le dispositif de commande (102), lorsqu'il est en mode d'entretien, est configuré pour envoyer le besoin d'entretien et l'identifiant à un dispositif sans fil d'un préposé à l'entretien.

7. Système de distribution selon la revendication 1, dans lequel le dispositif de commande (102), lorsqu'il est dans le mode d'entretien, est configuré pour communiquer avec l'au moins un de la pluralité de distributeurs (104) pour amener l'au moins un des distributeurs de la pluralité de distributeurs (104) à allumer un voyant sur l'au moins un de la pluralité de distributeurs (104).

8. Système de distribution selon la revendication 1, dans lequel le dispositif de commande (102), lorsqu'il est dans le mode d'entretien, est configuré pour communiquer avec l'au moins un de la pluralité de distributeurs (104) pour amener l'au moins un de la pluralité de distributeurs (104) à émettre un signal visuel indiquant qu'il a besoin d'entretien.

9. Système de distribution selon la revendication 1, dans lequel le dysfonctionnement électrique ou mécanique est un bourrage empêchant l'au moins un de la pluralité de distributeurs (104) d'effectuer la distribution ou de l'effectuer correctement.

10. Procédé comprenant :
la réception d'une demande au niveau d'un dispositif de commande (102) pour entrer dans un mode d'entretien, dans lequel le dispositif de commande (102) est situé dans une zone d'hygiène et a un mode de fonctionnement différent du mode d'entretien, dans lequel le dispositif de commande (102) a un haut-parleur (260) ;
l'entrée dans le mode d'entretien sur la base de la demande ;
dans le mode d'entretien, la fourniture d'une indication audible d'un besoin d'entretien d'un distributeur (104), dans lequel le distributeur (104) distribue un produit d'hygiène consommable, est situé dans la zone d'hygiène et a un identifiant ; et
dans lequel le besoin d'entretien est l'un parmi peu ou pas de produit d'hygiène consommable restant, un dysfonctionnement électrique ou mécanique et état de batterie faible,
**caractérisé en ce que** :
lorsqu'il est en mode d'entretien, la fourniture d'une indication audible d'un besoin d'entretien comprend la prononciation verbale à la fois du besoin d'entretien de l'au moins un de la pluralité de distributeurs (104) et de son identifiant par l'intermédiaire du haut-parleur (260) du dispositif de commande (102).

11. Procédé selon la revendication 10, comprenant en outre le fait que le dispositif de commande (102) communique un message au distributeur (104) pour amener le distributeur (104) à émettre un signal visuel du besoin d'entretien.

12. Procédé selon la revendication 11, dans lequel le signal visuel est un éclairage d'un voyant sur le distributeur.

13. Procédé selon la revendication 10, dans lequel la réception d'une demande au niveau d'un dispositif de commande (102) pour entrer dans un mode d'entretien comprend la réception d'un message provenant d'un dispositif sans fil d'un préposé à l'entretien qui donne l'instruction au dispositif de commande (102) d'entrer dans le mode d'entretien.

14. Procédé selon la revendication 10, dans lequel la réception d'une demande au niveau d'un dispositif de commande (102) pour entrer dans un mode d'entretien comprend la réception d'un ordre vocal qui donne l'instruction au dispositif de commande (102) d'entrer dans le mode d'entretien.
